# EUROPEAN PATENT SPECIFICATION

(11) **EP 2 699 220 B1**
(45) Date of publication and mention of the grant of the patent: **12.06.2019**
(21) Application number: 12774571.9
(22) Date of filing: 20.04.2012
(51) Int. Cl.: A23G 4/20, A23G 3/54, A23G 4/00, A23G 4/12, A61K 8/22, A61K 8/11, A61K 8/38, A61Q 11/00, A61K 8/73, A61K 8/81

(54) **IMPROVED STABILITY OF PEROXIDE IN ORAL CARE COMPOSITIONS**
VERBESSERTE STABILITÄT VON PEROXID IN MUNDPFLEGEZUSAMMENSETZUNGEN
STABILITÉ AMÉLIORÉE DE PEROXYDE DANS DES COMPOSITIONS DE SOIN BUCCAL

(30) Priority: 21.04.2011 US 201161477651 P
(43) Date of publication of application: 26.02.2014
(73) Proprietor: Intercontinental Great Brands LLC, East Hanover, NJ 07936 (US)
(72) Inventor: GEBRESELASSIE, Petros, Whitehouse Station, NJ 08889 (US); GAONKAR, Anilkumar, Ganapati, South Buffalo Grove, IL 60089 (US); CALAHAN, Allison, Evanston, IL 60202 (US); BOGHANI, Navroz, Budd Lake, NJ 07828 (US)
(74) Representative: Wilson Gunn
(86) International application number: PCT/US2012/034427
(87) International publication number: WO 2012/145611

(56) References cited:
- US-A- 2 290 862
- US-A- 5 059 416
- US-A1- 2006 177 383
- US-A1- 2006 204 613
- US-A1- 2008 063 747
- US-A1- 2009 214 445
- US-A1- 2010 104 689
- US-B1- 6 685 916

## Description

### Field of the Invention

The present invention is directed to a product and method for providing tooth whitening to a user.

In particular, the invention is directed to a chewing gum product including an encapsulated solid peroxide composition.

### Background of the Invention

Peroxides are known to provide tooth whitening effect to users. Due to its interaction with certain carriers, however, peroxides have typically been used as additives in gel compositions. There has been difficulty in providing peroxides to users in other forms that may be desirable to the user. For example, chewing gums have not typically been used to deliver peroxides due to the very short shelf life stability, since the peroxide reacts with gum base and quickly degrades.

US2009/21445A1 describes chewing gum compositions, some of which may be used as tooth whitening chewing gum compositions comprising coated particles containing a solid peroxide coated in polyvinyl acetate.

US2290862A discloses chewing gum compositions which may be used for tooth whitening, comprising alkaline earth metal peroxides mixed with hard hydrogenated fatty oils.

US5059416 discloses the use of multiple coatings of components in chewing gum compositions.

### Summary of the Invention

In one embodiment of the present invention, there is provided
a tooth whitening chewing gum composition comprising:
(a) a chewing gum base; and
(b) a plurality of active-containing particles, each of said active-containing particles comprising an inner region comprising a solid peroxide composition, a middle region comprising a first encapsulating material, and an outer region comprising a second encapsulating material,
wherein the first encapsulating material comprises polyvinyl acetate and the second encapsulating material comprises a cellulosic material; and
each of said active-containing particles comprises between 20- 50% by weight encapsulating material and between 50-80% by weight of peroxide.

In another embodiment, there is provided a method of whitening the teeth of a user, comprising the steps of:
(a) preparing a tooth whitening chewing gum composition comprising:
   (i) a chewing gum base; and
   (ii) a plurality of active-containing particles, each of said active-containing particles comprising an inner region comprising a solid peroxide composition, a middle region comprising a first encapsulating material, and an outer region comprising a second encapsulating material comprising a cellulosic material, wherein the first encapsulating material comprises polyvinyl acetate and the second encapsulating material comprises a cellulosic material; and wherein each of said active-containing particles comprises between 20-50% by weight of encapsulating material and between 50-80% by weight of peroxide;
(b) providing said chewing gum composition to a user; and
(c) said user chewing said chewing gum composition, wherein said chewing breaks said outer region and release said inner region into the mouth, thereby exposing said user's teeth to said peroxide composition.

In yet another embodiment, there is provided a method of whitening the teeth of a user as detailed in claim 17.

### Brief Description of the Drawings

Figure 1 is a graphical depiction of the results of one study demonstrating the stability of encapsulated peroxides.
Figure 2 is a graphical depiction of the results of one study demonstrating the amounts of carbamide peroxide recovered over various coating weights for encapsulated peroxides.
Figure 3 is a graphical depiction of the results of one study demonstrating the tooth whitening effect of products
Figure 4 is a graphical depiction of the results of a second study demonstrating the tooth whitening effect of products.
Figure 5 is a visual depiction of one embodiment of an encapsulated system.
Figure 6 is a visual depiction of one embodiment of a double encapsulation system according to the present invention.
Figure 7 is a chart depicting the results of one Example of the invention.

### Detailed Description of the Invention

The present invention is directed to chewing gum compositions with stain-removing properties for producing a whitening effect on dental surfaces that are treated with the same. The terms "stain removing" and "tooth whitening" are interchangeable and relate to the effect of whitening teeth surfaces. Such compositions are especially suitable for removing stains, which adhere to, or are entrapped in materials on, the surface of teeth and for preventing build-up of the stain entrapping material and stains on dental surfaces. The compositions of the present invention are meant to include whitening agents, which are not intentionally swallowed for purposes of systemic administration of therapeutic agents, but are retained in the oral cavity for a sufficient time to contact the dental surfaces for purposes of providing beneficial dental effects. In some embodiments, the compositions provided a foaming effect to the user, where the foam includes a tooth whitening agent and remains in contact with the tooth surface for a desirable period of time.

As used herein, the term "gum compositions" is intended to include any gum compositions, including "chewing gum" and "bubble gum."

As used herein, the term "confectionery" or "confectionery composition" includes compositions that do not include chewing gum or bubble gum. Such confectionery compositions can include, but are not limited to chocolate, compound coating, carob coating, cocoa butter, butter fat, hydrogenated vegetable fat, illipe butter, fondant including fondant-based creams, fudge, frappe, caramel, nougat, compressed tablet, candy floss (also known as cotton candy), marzipan, hard boiled candy, gummy candy, jelly beans, toffees, jellies including pectin-based gels, jams, preserves, butterscotch, nut brittles or croquant, candied fruit, marshmallow, pastilles, pralines or nougats, flour or starch confectionery, truffles, nonpareils, bon bons, after-dinner mints, fourres, nut pastes, peanut butter, chewing gum, kisses, angel kisses, montelimart, nougatine, fruit chews, Turkish delight, starch jellies, gelatin jellies, agar jellies, persipan, coconut paste, coconut ice, lozenges, cachous, creme paste, sugared nuts, sugared almonds, comfits, aniseed balls, licorice, licorice paste, chocolate spreads, chocolate crumb, truffles, gasified candy and combinations thereof.

Compositions for use herein may optionally include both a chewing gum portion and a confectionery region, which may be blended together or may be kept separate.

The compositions of the present invention may include an orally acceptable carrier, in an appropriate amount to accommodate the other components of the formulation. The term "orally acceptable carrier" refers to a vehicle capable of being mixed with the active components for delivery to the oral cavity for tooth whitening and cleaning purposes, and which will not cause harm to warm-blooded animals, including humans. The orally acceptable carriers further include those components of the composition that are capable of being comingled without interaction in a manner which would substantially reduce the composition's stability and/or efficacy for dental stain-removal in the oral cavity of warm-blooded animals, including humans, in accordance with the compositions and methods of the present invention.

The orally acceptable carriers of the present invention can include one or more compatible solid, liquid, slurry, gel, or encapsulating substances, which are suitable for oral administration. The carriers or excipients employed in the present invention may be in any form appropriate to the mode of delivery, for example, solutions, colloidal dispersions, emulsions, suspensions, rinses, gels, foams, powders, solids, and the like, and can include conventional components of toothpastes (including gels), mouthwashes and rinses, tooth powders, tooth hardeners, antiplaque compositions, mouth sprays, chewing gums, lozenges, and confectioneries. Carriers suitable for the preparation of compositions of the present invention are well known in the art. Their selection will depend on secondary considerations like taste, cost, shelf stability and the like.

As will be described in further detail below, an action that breaks the encapsulated peroxide is useful in the invention to provide the peroxide to the teeth. Such actions include chewing, brushing, and the like.

The chewing gum compositions of the present invention may be coated or uncoated, and be in the form of slabs, sticks, pellets, balls and the like. The composition of the different forms of the chewing gum compositions will be similar but may vary with regard to the ratio of the ingredients. For example, coated gum compositions may contain a lower percentage of softeners. Pellets and balls have a small chewing gum core, which has been coated with either a sugar solution or a sugarless solution to create the hard shell. Slabs and sticks are usually formulated to be softer in texture than the chewing gum core. Especially preferred embodiments include a composition in the form of a chewing gum slab.

Chewing gum compositions of the present invention include a gum base and most of the other typical chewing composition components, such as sweeteners, softeners, flavorants and the like, which will be described in further detail below. Desirably, at least one stain-removing agent, or tooth whitening agent, is employed in the inventive compositions.

The stain removing agent is a solid peroxide composition. Solid peroxide compositions are useful in the present invention due to their ability to be encapsulated easily and incorporated into a chewing gum base with relative ease. The most preferred peroxide composition includes carbamide peroxide or urea peroxide, however, other compositions may be used, including sodium percarbonate, peroxydone, calcium peroxide and perbenzoic acid. Due to the ability of such peroxides to break down gum base quickly, it is particularly useful that there be a separation between the gum base and the peroxide composition. For this reason, it is useful that the peroxide composition be encapsulated in a suitable encapsulant. The amount of peroxide composition in the chewing gum, the amount of encapsulating material in the chewing gum, and the relative ratios of peroxide to encapsulant are each of importance in the invention, as will be explained in further detail below. It is useful that there be enough of the encapsulant to provide an effective barrier between the peroxide and the gum base, but not too much encapsulant so as to reduce the whitening effectiveness of the chewing gum or the mouth feel. In addition, as will be explained below, the present inventors have discovered the optimal balance between encapsulant and peroxide so as to provide an effective barrier while also improving processing ability of the product.

As will be explained in further detail below, the compositions of the present invention may include any number of additives or components to provide a desired effect. Types of additives or ingredients, which may be included in the present compositions, include one or more desirable stain-removing agents as provided herein. The inventive compositions may also include a component selected from the following: elastomers, elastomer solvents, waxes, emulsifiers, plasticizers, softeners, dispersing agents, sweeteners, flavorants, humectants, active agents, cooling agents, warming agents, other tooth whitening agents, colorants, bulking agents, fillers and combinations thereof.

In some embodiments, an additional active agent can be a fluoride compound or an antibacterial compound. For example, a known antibacterial compound is triclosan. Other compounds include, for example, iso-propyl-methyl-phenol, dextrase, chlorophyll, sodium copper chlorophyll, flavonoid, mutanase, lysozyme, amylase, protease, lytic enzymes, superoxide dismutaze, epsilon aminocaproic acid, aluminum allantoin, aluminum chloro-hydroxy-allantoin, dihydro-cholestanol, bisabolol, glycerophosphate, water soluble inorganic phosphorylated compounds; fluorides such as sodium fluoride, sodium monofluorophosphate, stannous fluoride, zinc citrate, copper gluconate, chlorhexidine gluconate, vitamins such as vitamin A, vitamin C, vitamin E, vitamin B6 and pantothenate; amino acids such as glycin, lysine and histidine; sodium bicarbonate, potassium nitrate, sarcosinate; polyphenol compounds such as catechins; naficillin, oxacillin, vancomycin, clindamycin, erythromycin, trimethoprim-sulphamethoxazole, rifampin, ciprofloxacin, broad spectrum penicillin, amoxicillin, gentamicin, ceftriazoxone, cefotaxime, chloramphenicol, clavunate, sulbactam, probenecid, doxycycline, spectinomycin, cefixime, penicillin G, minocycline, .beta.-lactamase inhibitors; meziocillin, piperacillin, aztreonam, norfloxacin, trimethoprim, ceftazidime, dapsone, halogenated diphenyl ethers, phenolic compounds including phenol and its homologs, mono and poly-alkyl and aromatic halophenols, resorcinol and its derivatives, bisphenolic compounds and halogenated salicylanilides, benzoic esters, and halogenated carbanilides, magnolia bark extracts, honokiol, magnolol, morin, geraniol, hop extracts, lithospermi radix (gromwell), myristicae semen (nutmeg), arecae semen (betel nut), artemisia princeps (mugwort), phellodendri cortex (phellodendron bark), moutan cortex (moutan bark), sctellariae radix (scutellaria root), rhei rhizoma (rhubarb), chrysanthemum indicum (wild chrysanthemum), leonuri herba (leonurus sibiricus), Aesculus hippocastanum, Aesculus turbinata, rose and Zanthoxyli Fructus.

The surface active agents may include, for example, ionic surface active agents, cationic surface active agents, ampholytic surface active agents and nonionic surface active agents.

Specifically, such agents may include, for example, an alkyl sulfate, an alkyl benzene sulfonate, a sucrose fatty acid ester, a lactose fatty acid ester, a salt of lauroyl sarcosinc, salts of fatty acids such as Sodium Stearate N-acyl glutamic acid, alpha-olefin sulfonate, 2-alkyl-N-carboxy-N-hydroxyethylimidazorium betaine, a salt of N-acyltaurine, alkylol amide, polyoxyethylene sorbitan fatty acid ester, polyoxyethylene hydrogenated castor oil and fatty acid ester thereof, polyglycerin fatty acid ester, sorbitan fatty acid ester, fatty acid ester, polyethyiene glycol fatty acid ester, and propylene glycol fatty acid ester.

Moreover, in some embodiments a film-forming polymer may be included in the compositions of the present invention. For example, a film-form in polymer may be a synthetic anionic polymeric polycarboxylate (SAPP), such a PVM/MA copolymer (Gantrez S-97, GAF Corp.). Such polymers are described in U.S. Patent Nos. 5,334,375 and 5,505,933. SAPP's have previously been described as being useful for dentin sensitivity reduction. Moreover, SAPP's have previously been described as antibacterial-enhancing agents, Which enhance delivery of an antibacterial agent to oral surfaces, and which enhance the retention of the antibacterial agent on oral surfaces. It is well within the contemplation of the present invention that film-forming polymers, such as PVM/MA copolymer, may be employed in the compositions of the present invention as a means of further reducing stain formation.

Gum compositions according to the present invention may further include a chelator, such as a polyphosphate. For example, sodium tripolyphosphate may be used if desired. In one particular embodiment, sodium tripolyphosphate may be used in an amount of from 1 - 10% by weight of the composition, and more particularly 3-6%, and most desirably 4% by weight of the composition. The polyphosphate may be present in free or encapsulated form.

The chewing gum compositions include a gum base as part of the carrier. The gum base may be present in an amount of 20 to 40% by weight of the total composition. It may include any component known in the chewing gum art. For example, the gum base may include sweeteners, elastomers, bulking agents, waxes, elastomer solvents, emulsifiers, plasticizers, fillers, mixtures thereof and may include a desirable stain-removing agent(s) as provided herein.

The elastomers (rubbers) employed in the gum base will vary greatly depending upon various factors such as the type of gum base desired, the consistency of gum composition desired and the other components used in the composition to make the final chewing gum product. The elastomer may be any water-insoluble polymer known in the art, and includes those gum polymers utilized for chewing gums and bubble gums. Illustrative examples of suitable polymers in gum bases include both natural and synthetic elastomers. For example, those polymers which are suitable in gum base compositions include, without limitation, natural substances (of vegetable origin) such as chicle, natural rubber, crown gum, nispero, rosidinha, jelutong, perillo, niger gutta, tunu, balata, guttapercha, lechi capsi, sorva, gutta kay, and the like, and mixtures thereof. Examples of synthetic elastomers include, without limitation, styrene-butadiene copolymers (SBR), polyisobutylene, isobutylene-isoprene copolymers, polyethylene, polyvinyl acetate and the like, and mixtures thereof.

The amount of elastomer employed in the gum base may vary depending upon various factors such as the type of gum base used, the consistency of the gum composition desired and the other components used in the composition to make the final chewing gum product. In general, the elastomer will be present in the gum base in an amount from 10% to 60% by weight of the gum base, desirably from 35% to 40% by weight.

When a wax is present in the gum base, it softens the polymeric elastomer mixture and improves the elasticity of the gum base. The waxes employed will have a melting point below 60°C., and preferably between 45°C and 55°C. The low melting wax may be a paraffin wax. The wax may be present in the gum base in an amount from 1% to 10%, and preferably from 7% to 9.5%, by weight of the gum base.

In addition to the low melting point waxes, waxes having a higher melting point may be used in the gum base in amounts up to 5%, by weight of the gum base. Such high melting waxes include beeswax, vegetable wax, candelilla wax, carnuba wax, most petroleum waxes, and the like, and mixtures thereof.

In addition to the components set out above, the product, whether it be a chewing gum product or other product, may include a variety of other ingredients, such as components selected from the following: sweetening agents (sweeteners), plasticizers, softeners, emulsifiers, waxes, fillers, bulking agents (carriers, extenders, bulk sweeteners), mineral adjuvants, flavoring agents (flavors, flavorings), coloring agents (colorants, colorings), antioxidants, acidulants, thickeners, medicaments, and the like, and mixtures thereof. Some of these additives may serve more than one purpose. For example, in sugarless gum compositions, a sweetener, such as maltitol or other sugar alcohol, may also function as a bulking agent.

A gum base may contain elastomer solvents to aid in softening the elastomer component. Such elastomer solvents may include those elastomer solvents known in the art, for example, terpinene resins such as polymers of alpha-pinene or beta-pinene, methyl, glycerol and pentaerythritol esters of rosins and modified rosins and gums such as hydrogenated, dimerized and polymerized rosins, and mixtures thereof. Examples of elastomer solvents suitable for use herein may include the pentaerythritol ester of partially hydrogenated wood and gum rosin, the pentaerythritol ester of wood and gum rosin, the glycerol ester of wood rosin, the glycerol ester of partially dimerized wood and gum rosin, the glycerol ester of polymerized wood and gum rosin, the glycerol ester of tall oil rosin, the glycerol ester of wood and gum rosin and the partially hydrogenated wood and gum rosin and the partially hydrogenated methyl ester of wood and rosin, and the like, and mixtures thereof. The elastomer solvent may be employed in the gum base in amounts from 2% to 15%, and preferably from 7% to 11%, by weight of the gum base.

The gum base may also include emulsifiers, which aid in dispersing any immiscible components into a single stable system. The emulsifiers useful in this invention include glyceryl monostearate, lecithin, fatty acid monoglycerides, diglycerides, polysorbates, sugar esters, polyglycerol esters, propylene glycol monostearate, and the like, and mixtures thereof. The emulsifier may be employed in amounts from 0.1% to 15%, and more specifically, from 0.5% to 11%, by weight of the gum base.

The gum base may also include plasticizers or softeners to provide a variety of desirable textures and consistency properties. Because of the low molecular weight of these ingredients, the plasticizers and softeners are able to penetrate the fundamental structure of the gum base making it plastic and less viscous. Useful plasticizers and softeners include lanolin, palmitic acid, oleic acid, stearic acid, sodium stearate, potassium stearate, glyceryl triacetate, glyceryl lecithin, glyceryl monostearate, propylene glycol monostearate, distilled monoglycerides, acetylated monoglyceride, glycerin, and the like, and mixtures thereof. Waxes, for example, natural and synthetic waxes, hydrogenated vegetable oils, petroleum waxes such as polyurethane waxes, polyethylene waxes, paraffin waxes, microcrystalline waxes, fatty waxes, sorbitan monostearate, tallow, propylene glycol, mixtures thereof, and the like, may also be incorporated into the gum base. Plasticizers also include are the hydrogenated vegetable oils and include soybean oil and cottonseed oil which may be employed alone or in combination. These plasticizers provide the gum base with good texture and soft chew characteristics. The plasticizers and softeners are generally employed in the gum base in amounts up to 20% by weight of the gum base, and more specifically in amounts from 9% to 17%, by weight of the gum base. The plasticizers and softeners may be used in amounts of 0.5% to t 10% by weight of the finished product.

Anhydrous glycerin may also be employed as a softening agent, such as the commercially available United States Pharmacopeia (USP) grade. Glycerin is a syrupy liquid with a sweet warm taste and has a sweetness of 60% of that of cane sugar. Because glycerin is hygroscopic, the anhydrous glycerin may be maintained under anhydrous conditions throughout the preparation of the chewing gum composition.

Although softeners may be present to modify the texture of the gum composition, they may be present in reduced amounts as compared to typical gum compositions. For example, they may be present from 0.5 to 10% by weight based on the total weight of the composition, or they may not be present in the composition, since the surfactant can act as a softener.

The gum base useful in this invention may also include effective amounts of bulking agents such as mineral adjuvants Which may serve as fillers and textural agents. Useful mineral adjuvants include calcium carbonate, magnesium carbonate, alumina, aluminum hydroxide, aluminum silicate, talc, tricalcium phosphate, dicalcium phosphate, calcium sulfate and the like, and mixtures thereof. These fillers or adjuvants may be used in the gum base compositions in various amounts. Preferably the amount of filler, when used, will be present in an amount from 10% to 40%, and desirably from 20% to 30%, by weight of the gum base.

A variety of traditional ingredients may be optionally included in the gum base in effective amounts such as coloring agents, antioxidants, preservatives, flavoring agents, and the like. For example, titanium dioxide and other dyes suitable for food, drug and cosmetic applications, known as F. D. & C. dyes, may be utilized. An anti-oxidant such as butylated hydroxytoluene (BHT), butylated hydroxyanisole (BHA), tocopherols, propyl gallate, and mixtures thereof may also be included. Other conventional chewing gum additives known to one having ordinary skill in the chewing gum mi may also be used in the gum base.

The plasticizers, softening agents, mineral adjuvants, waxes and antioxidants discussed above, as being suitable for use in the gum base, may also be used in the chewing gum composition. Examples of other conventional additives which may be used include emulsifiers, such as lecithin and glyceryl monostearate, thickeners, used alone or in combination with other softeners, such as methyl cellulose, alginates, carrageenan, xanthan gum, gelatin, carob, tragacanth, locust bean, and carboxy methyl cellulose, acidulants such as malic acid, adipic acid, citric acid, tartaric acid, fumaric acid, and mixtures thereof, and fillers, such as those discussed above under the category of mineral adjuvants.

Compositions of the present invention may desirably include a sweetener or a sugar bulking agent. In some embodiments, the composition may include a suitable sugar bulking agent.

Suitable sugar bulking agents include monosaccharides, disaccharides and polysaccharides such as xylose, ribulose, glucose (dextrose), mannose, galactose, fructose (levulose), sucrose (sugar), maltose, invert sugar, partially hydrolyzed starch and corn syrup solids, and mixtures thereof. For example, the product may include a specific polyol composition including at least one polyol that is from 30% to 80% by weight of the sweetening
composition, and desirably from 50% to 60%. The polyol composition may include any polyol known in the art including, but not limited to maltitol, sorbitol, erythritol, xylitol, mannitol, galactitol, isomalt, lactitol and combinations thereof. Lycasin which is a hydrogenated starch hydrolysate including sorbitol and maltitol, may also be used.

Maltitol is a sweet, water-soluble sugar alcohol useful as a bulking agent in the preparation of beverages and foodstuffs and is more fully described in U.S. Pat. No. 3,708,396. Maltitol is made by hydrogenation of maltose which is the most common reducing disaccharide and is found in starch and other natural products. Useful sweetening compositions include a mixture of isomalt and maltitol, which provides a hydroscopic sweetening composition.

The sweetener or sweetening composition may include one or more different polyols which may be derived from a genetically modified organism ("GMO") or GMO free source. For example, the maltitol may be GMO free maltitol or provided by a hydrogenated starch hydrolysate.

Suitable hydrogenated starch hydrolysates include those disclosed in U.S. Pat. Nos. 25,959, 3,356,811, 4,279,931 and various hydrogenated glucose syrups and/or powders which contain sorbitol, hydrogenated disaccharides, hydrogenated higher polysaccharides, or mixtures thereof. Hydrogenated starch hydrolysates are primarily prepared by the controlled catalytic hydrogenation of corn syrups. The resulting hydrogenated starch hydrolysates are mixtures of monomeric, dimeric, and polymeric saccharides. The ratios of these different saccharides give different hydrogenated starch hydrolysates different properties. Mixtures of hydrogenated starch hydrolysates, such as LYCASIN, a commercially available product manufactured by Roquette Freres of France, and HYSTAR, a commercially available product manufactured by Lonza, Inc., of Fairlawn, N.J., are also useful.

The sweetening agents used may be selected from a wide range of materials including water-soluble sweeteners, water-soluble artificial sweeteners, water-soluble sweeteners derived from naturally occurring water-soluble sweeteners, dipeptide based sweeteners, and protein based sweeteners, including mixtures thereof. Without being limited to particular sweeteners, representative categories and examples include:
(a) water-soluble sugar alcohols such as sorbitol, mannitol, maltitol, xylitol, erythritol and L-aminodicarboxylic acid aminoalkenoic acid ester amides, such as those disclosed in U.S. Pat. No. 4,619,834, and mixtures
   thereof;
(b) water-soluble artificial sweeteners such as soluble saccharin salts, i.e., sodium or calcium saccharin salts, cyclamate salts, the sodium, ammonium or calcium salt of 3,4-dihydro-6-methyl-1,2,3-oxathiazine-4-one-2,2-dioxide, the potassium salt of 3,4-dihydro-6-methyl-1,2,3-oxathiazine-4-one-2,2-dioxide (Acesulfame-K), the free acid form of saccharin, and mixtures thereof;
(c) dipeptide based sweeteners, such as L-aspartic acid derived sweeteners, such as L-aspartyl-L-phenylalanine methyl ester (Aspartame) and materials described in U.S. Pat. No. 3,492,131, L-alphaaspartyl-N-(2,2,4,4-tetramethyl-3-thietanyl)-D-alaninamide hydrate (Alitame), N-[N-(3,3-dimethylbutyl)-L-aspartyl]-L-phenylalanine 1-methyl ester (Neotame), methyl esters of L-aspartyl-L-phenylglycerine and L-aspartyl-L-2,5-dihydrophenyl-glycine, L-aspartyl-2,5-dihydro-L-phenylalanine; L-aspartyl-L-(1-cyclohexen)-alanine, and mixtures thereof;
(d) water-soluble sweeteners derived from naturally occurring water-soluble sweeteners, such as chlorinated derivatives of ordinary sugar (sucrose), e.g., chlorodeoxysugar derivatives such as derivatives of chlorodeoxysucrose or chlorodeoxygalactosucrose, known, for example, under the product designation of Sucralose; examples of chlorodeoxysucrose and chlorodeoxygalactosucrose derivatives include but are not limited to: 1-chloro-1'-deoxysucrose; 4-chloro-4-deoxy-alpha-D-galactopyranosyl-alpha-D-fructofuranoside, or 4-chloro-4-deoxygalactosucrose; 4-chloro-4-deoxy-alpha-D-galactopyranosyl-1-chloro-1-deoxy-beta-D-fructo-furanoside, or 4,1'-dichloro-4,1'-dideoxygalactosucrose; 1',6'-dichloro 1',6'-dideoxysucrose; 4-chloro-4-deoxy-alpha-D-galactopyranosyl-1,6-dichloro-1,6-dideoxy-beta-D-fructo furanoside, or 4,1',6'-trichloro-4,1',6'-trideoxygalactosucrose; 4,6-dichloro-4,6-dideoxy-alpha-D-galactopyranosyl-6-chloro-6-deoxy-beta-D-fructofuranoside, or 4,6,6'-trichloro-4,6,6'-trideoxygalactosucrose; 6,1', 6'-trichloro-6,1',6'-trideoxysucrose; 4,6-dichloro-4,6-dideoxy-alpha-D-galacto-pyranosyl-1,6 dichloro-1,6-dideoxy-beta -D-fructofuranoside, or 4,6,1',6'-tetrachloro4,6,1',6'-tetradeoxygalacto-sucrose; and 4,6, 1',6'-tetradeoxy-sucrose, and mixtures thereof;
(e) protein based sweeteners such as thaumatococcus danielli (Thaumatin I and II) and talin;
(f) the sweetener monatin (2-hydroxy-2-(indol-3-ylmethyl)-4-aminoglutaric acid) and its derivatives; and
(g) the sweeteners rebaudioside A, rebaudioside B, rebaudioside C, rebaudioside D, rebaudioside E, rebaudioside F, dulcoside A, dulcoside B, rubusoside, stevia, stevioside, mogroside IV, mogroside V, Luo Han Quo sweetener (sometimes also referred to as "Lo han kuo "or "Lo han quo"), siamenoside, monatin and its salts (monatin SS, RR, RS, SR), glycyrrhizic acid and its salts, hemandulcin, phyllodulcin, glycyphyllin, dihydroflavenol, dihydrochalcones, phloridzin, trilobatin, baiyunoside, osladin, polypodoside A, pterocaryoside A, pterocaryoside B, mukurozioside, phlomisoside I, periandrin I, abrusoside A, and cyclocarioside I. Such high intensity sweeteners can be used at any suitable purity level. Additionally, the purification of rebaudioside A by crystallization can result in the formation of at least three different polymorphs: a rebaudioside A hydrate; an anhydrous rebaudioside A; and a rebaudioside A solvate. In addition to the at least three polymorph forms of rebaudioside A, the purification of rebaudioside A may result in the formation of an amorphous form of rebaudioside A.

Preferably, the high intensity sweetening agent comprises the potassium salt of 3,4-dihydro-6-methyl-1,2,3-oxathiazine-4-one-2,2-dioxide, L-aspartyl-L-phenylalanine methyl ester, L-alpha-aspartyl-N-(2,2,4,4-tetramethyl-3-thietanyl)-D-alaninamide hydrate, N-[N-(3,3-dimethylbutyl)-L-aspartyl]-L-phenylalanine 1-methyl ester, chlorinated derivatives of sucrose, thaumatin, monatin, mogrosides, or a combination comprising at least one of the foregoing high intensity sweetening agents. More preferably the chewing gum composition comprises a high intensity sweetening agent comprising sucralose and acesulfame K. Intense sweetening agents may be used in many distinct physical forms well-known in the art to provide an initial burst of sweetness and/or a prolonged sensation of sweetness. Without being limited thereto, such physical forms include free forms, such as spray dried, powdered, beaded forms, encapsulated forms, and mixtures thereof.

The most desirable sweeteners include sucralose, Acesulfame potassium, sorbitol, mannitol, isomalt, and combinations thereof. The sweetener or sweetening composition may be present in an amount of from 0.5% to 70% by weight of the finished product.

In general, an effective amount of sweetener may be utilized to provide the level of sweetness desired, and this amount may vary with the sweetener selected. The sweetener may be used in conjunction with the peroxide, to provide a taste masking effect to the user. The exact range of amounts for each type of sweetener may be selected by those skilled in the art.

In one particularly preferred embodiment, the inventive product includes sucralose in an amount of from 1-10% by weight of the product, and more particularly in an amount of 3% to 5% by weight of the product. As will be described in more detail below,
the use of sucralose is preferred due to its lack of substantial interaction with the peroxides of the present invention. For example, certain sweeteners, such as aspartame, may have a tendency to react with peroxide, thus reducing the stain removing effect that the peroxide may have.

The present inventors have compared the action of certain sweeteners on the effect with carbamide peroxide, with surprising results. Carbamide peroxide was stored with three sweeteners, and stored for four weeks at 25°C to determine stability. The three sweeteners included aspartame, acesulfame potassium and sucralose. The percentage of carbamide peroxide recovered after four weeks was measured. The results are set forth in Table 1 below:

**Table 1 - Percentage of Carbamide Peroxide Recovered**

| **Sweetener** | % Carbamide Peroxide recovered after 4 weeks of aging with sweetener at 25C |
|---|---|
| Aspartame | 78 |
| Ace- K | 92 |
| Sucralose | 100 |

As can be seen, the combination of sucralose and carbamide peroxide was found to leave 100% of the peroxide remaining after four weeks, demonstrating the clear lack of reaction between the two compounds. Ace-K was found to leave 92% of the peroxide, which is considered to be an acceptable level. The use of aspartame demonstrated a significant reduction in the available carbamide peroxide after four weeks, rendering it the least effective of the three sweeteners tested.

Chewing gum compositions of the present invention may include flavors or flavoring agents. Useful flavoring agents include those flavors known to the skilled artisan, such as natural and artificial flavors. These flavorings may be chosen from synthetic flavor oils and flavoring aromatics and/or oils, oleoresins and extracts derived from plants, leaves, flowers, fruits, and so forth, and combinations thereof. Nonlimiting representative flavor oils include spearmint oil, cinnamon oil, oil of wintergreen (methyl salicylate), peppermint oil, clove oil, bay oil, anise oil, eucalyptus oil, thyme oil, cedar leaf oil, oil of nutmeg, allspice, oil of sage, mace, oil of bitter almonds, and cassia oil. Also useful flavorings are artificial, natural and synthetic fruit flavors such as vanilla, and citrus oils including lemon, orange, lime, grapefruit, and fruit essences including apple, pear, peach, grape, strawberry, raspberry, cherry, plum, pineapple, apricot and so forth. These flavoring agents may be used in liquid or solid form and may be used individually or in admixture. Commonly used flavors include mints such as peppermint, menthol, spearmint, artificial vanilla, cinnamon derivatives, and various fruit flavors, whether employed individually or in admixture.

Other useful flavorings include aldehydes and esters such as cinnamyl acetate, cinnamaldehyde, citral diethylacetal, dihydrocarvyl acetate, eugenyl formate, p-methylamisol, and so forth may be used. Generally any flavoring or food additive such as those described in Chemicals Used in Food Processing, publication 1274, pages 63-258, by the National Academy of Sciences, may be used.

Further examples of aldehyde flavorings include but are not limited to acetaldehyde (apple), benzaldehyde (cherry, almond), anisic aldehyde (licorice, anise), cinnamic aldehyde (cinnamon), citral, i.e., alpha-citral (lemon, lime), neral, i.e., beta-citral (lemon, lime), decanal (orange, lemon), ethyl vanillin (vanilla, cream), heliotrope, i.e., piperonal (vanilla, cream), vanillin (vanilla, cream), alpha-amyl cinnamaldehyde (spicy fruity flavors), butyraldehyde (butter, cheese), valeraldehyde (butter, cheese), citronellal (modifies, many types), decanal (citrus fruits), aldehyde C-8 (citrus fruits), aldehyde C-9 (citrus fruits), aldehyde C-12 (citrus fruits), 2-ethyl butyraldehyde (berry fruits), hexenal, i.e., trans-2 (berry fruits), tolyl aldehyde (cherry, almond), veratraldehyde (vanilla), 2,6-dimethyl-5-heptenal, i.e., melonal (melon), 2,6-dimethyloctanal (green fruit), and 2-dodecenal (citrus, mandarin), cherry, grape, strawberry shortcake, and mixtures thereof.

In some embodiments, the flavoring agent may be employed in either liquid form and/or dried form. When employed in the latter form, suitable drying means such as spray drying the oil may be used. Alternatively, the flavoring agent may be absorbed onto water soluble materials, such as cellulose, starch, sugar, maltodextrin, gum arabic and so forth or may be encapsulated. The actual techniques for preparing such dried forms are well-known.

In some embodiments, the flavoring agents may be used in many distinct physical forms well-known in the art to provide an initial burst of flavor and/or a prolonged sensation of flavor. Without being limited thereto, such physical forms include free forms, such as spray dried, powdered, beaded forms, encapsulated forms, and mixtures thereof.

The amount of flavoring agent employed herein may be a matter of preference subject to such factors as the type of final composition, the individual flavor, the gum base employed, and the strength of flavor desired. Thus, the amount of flavoring may be varied in order to obtain the result desired in the final product and such variations are within the capabilities of those skilled in the art without the need for undue experimentation. In gum compositions, the flavoring agent is generally present in amounts from 0.02% to 5%, and more specifically from 0.1% to 2%, and even more specifically, from about 0.8% to 1.8%, by weight of the composition.

Coloring agents may be used in amounts effective to produce the desired color. The coloring agents may include pigments which may be incorporated in amounts up to 6%, by weight of the composition. For example, titanium dioxide may be incorporated in amounts up to 2%, and preferably less than 1%, by weight of the gum composition. The colorants may also include natural food colors and dyes suitable for food, drug and cosmetic applications. These colorants are known as F.D.& C. dyes and lakes. The materials acceptable for the foregoing uses are preferably water-soluble. Illustrative nonlimiting examples include the indigoid dye known as F.D.& C. Blue No.2, which is the disodium salt of 5,5-indigotindisulfonic acid. Similarly, the dye known as F.D.& C. Green No.1 comprises a triphenylmethane dye and is the monosodium salt of 4-[4-(N-ethyl-p-sulfoniumbenzylamino) diphenylmethylene]-[1-(N-ethyl-N-p-sulfoniumbenzyl)-delta-2,5-cyclohexadieneimine]. A full recitation of all F.D.& C. colorants and their corresponding chemical structures may be found in the Kirk-Othmer Encyclopedia of Chemical Technology, 3rd Edition, in volume 5 at pages 857-884.

Suitable oils and fats usable in compositions include partially hydrogenated vegetable or animal fats, such as coconut oil, palm kernel oil, beef tallow, and lard, among others. These ingredients when used are generally present in amounts up to about 7%, and preferably up to about 3.5%, by weight of the composition.

In some embodiments, the tooth whitening compositions of the present invention include an abrasive agent. Suitable abrasives include silicas, aluminas, phosphates, carbonates and combinations thereof. In some embodiments, the abrasive agent is a silica selected from: precipitated silica, silica gels and combinations thereof. Moreover, in some embodiments the abrasive agent is selected from the following: calcium carbonate, sodium bicarbonate, sodium metaphosphate, potassium metaphosphate, tricalcium phosphate, dehydrated dicalcium phosphate and combinations thereof.

The abrasive polishing material contemplated for use in the compositions of the present invention can be any material which does not excessively abrade dentin. However, silica dental abrasives have unique benefits of exceptional dental cleaning and polishing performance without unduly abrading tooth enamel or dentin.

The silica abrasive polishing materials herein, as well as other abrasives, generally have an average particle size ranging between 0.1 to 30 microns, and preferably from 5 to 15 microns. The abrasive can be precipitated silica or silica gels such as the silica xerogels described in U.S. Patent No. 3,538,230 to Pader, et al. and U.S. Patent No. 3,862,307 to DiGiulio. Preferred are
the silica xerogels marketed under the trade name "Syloid" by the W.R. Grace & Company, Davison Chemical Division. Also preferred are the precipitated silica materials, such as those marketed by the J.M. Huber Corporation under the trade name "Zeodent", particularly the silica carrying the designation "Zeodent 119". The types of silica dental abrasives useful in the present invention are described in detail in U.S. Patent No. 4,340,583 to Wason.

Silica abrasives are described in U.S. Patent Application Serial Nos. 08/434,147 and 08/434,149, both filed May 2, 1995.

In some embodiments, the abrasive is present in amounts from 0.1 to 30% by weight of the composition. The abrasive agent may be more typically employed in amounts from 0.5 to 5% by weight of the total composition. The abrasive in a toothpaste compositions of this invention, for example, may generally be present at a level of from
0.5% to 10% by weight of the composition. Chewing gum compositions of the present invention may contain from 1% to 6% of abrasive, by weight of the composition.

The silica used to prepare a chewing gum composition, for example, is differentiated by means of its oil absorption value, having oil absorption value of less than 100 cc/100g, and preferably in the range of from 45 cc/100g silica to less than 70 cc/100g silica. Silica particularly useful in the practice of the present invention is marketed under the trade designation SYLODENT XWA GRACE Davison Co., Columbia, DS 21044. An example of such silica is SYLODENT XWA 150, a silica precipitate having a water content of 4.7% by weight averaging from 7 to 11 microns in diameter, having an Einlehner Hardness of 5, a BET surface area of 390 m²/g of silica, an oil absorption of less than 70 cm³/100g of silica. This silica exhibits low abrasiveness to tooth enamel.

The silica abrasive can be used as the sole abrasive in preparing a chewing gum composition, or it may be used in combination with other known abrasives or polishing agents, including calcium carbonate, sodium bicarbonate, sodium metaphosphate, potassium metaphosphate, tricalcium phosphate, dehydrated dicalcium phosphate, or other siliceous materials, or combinations thereof.

In some embodiments, the total quantity of abrasive silica present in the composition is at a concentration of from 0.1 to 20% by weight of the chewing gum composition.

Moreover, in some embodiments, the total quantity of abrasive silica present in a chewing gum composition of the present invention, for example, is from 0.5% to 5% by weight.

In some embodiments, confectionery or chewing gum pieces may be coated with an aqueous coating composition, which may be applied by any method known in the art. The coating composition may be present in an amount from 25% to 35% by weight of the total piece, more specifically 30% by weight of the piece.

An outer coating may be hard or crunchy. Typically, the outer coating may include sorbitol, maltitol, xylitol, isomalt, and other crystallizable polyols; sucrose may also be used. Flavors may also be added to yield unique product characteristics. Moreover, the outer coating may include one or more of the stain-removing agents provided herein. Alternatively, the product may include a coating that is particulate or powder in form, such as a dusted coating.

The coating, if present, may include several opaque layers, such that the chewing gum composition is not visible through the coating itself, which can optionally be covered with a further one or more transparent layers for aesthetic, textural and protective purposes. The outer coating may also contain small amounts of water and gum arabic. The coating can be further coated with wax. The coating may be applied in a conventional manner by successive applications of a coating solution, with drying in between each coat. As the coating dries it usually becomes opaque and is usually white, though other colorants may be added. A polyol coating can be further coated with wax. The coating can further include colored flakes or speckles.

If the composition comprises a coating, it is possible that one or more oral care actives, such as stain removing agents or breath freshening agents, can be dispersed throughout the coating. This may be preferred if one or more oral care actives is incompatible in a single phase composition with another active.

Moreover, it is well within the contemplation of the present invention that providing one or more of the stain-removing agents in the coating can enhance the stain-removing efficacy of the total composition. For example, as described above, the mechanical abrasion may be initially enhanced by providing an abrasive in the coating layer. Chemical cleaning effects are also enhanced as a result.

Additives, such as physiological cooling agents, throat-soothing agents, spices, warming agents, tooth-whitening agents, breath-freshening agents, vitamins minerals, caffeine, drugs and other actives may be included in any or all portions of the stain removing composition. Such components may be used in amounts sufficient to achieve their intended effects.

With respect to cooling agents, a variety of well known cooling agents may be employed. For example, among the useful cooling agents are included menthol, xylitol, menthane, menthone, menthyl acetate, menthyl salicylate, N,2,3-trimethyl-2-isopropyl butanamide (WS-23), N-ethyl-p-menthane-3-carboxamide (WS-3), menthyl succinate, 3, 1-menthoxypropane 1,2-diol, among others. These and other suitable cooling agents are further described in the following US. patents:
U.S. 4,230,688 and 4,032,661 to Rowsell et al.; 4,459,425 to Amano et al.; 4,136,163 to Watson et al.; and 5,266,592 to Grub et al. These cooling agents may be present in any region of the product. Cooling agents, when used in the outer coating composition for the product, are generally present in amount of 0.01% to 1.0%. When used in the other portions of a chewing gum, such as a gum region or in a center fill region, they may be present in amounts of 0.001 to 10% by weight of the total chewing gum.

Warming components may be selected from a wide variety of compounds known to provide the sensory signal of warming to the user. These compounds offer the perceived sensation of warmth, particularly in the oral cavity, and often enhance the perception of flavors, sweeteners and other organoleptic components, Among the useful warming compounds included are vanillyl alcohol n-butylether (TK-1000) supplied by Takasago Perfumary Company Limited, Tokyo, Japan, vanillyl alcohol n-propylether, vanillyl alcohol isopropylether, vanillyl alcohol isobutylether, vanillyl alcohol n-aminoether, vanillyl alcohol isoamyleather, vanillyl alcohol n-hexyleather, vanillyl alcohol methylether, vanillyl alcohol ethyleather, gingerol shogaol, paradol, zingerone, capsaicin, dihydrocapsaicin, nordihydrocapsaicin, homocapsaicin, homodihydrocapsaicin, ethanol, isopropyl alcohol, iso-amylalcohol, benzyl alcohol, glycerine, and combinations thereof The chewing gum compositions of the present invention desirably include a peroxide composition, and most desirably a solid peroxide composition. Solid peroxide compositions are preferred due to their ease of being coated with an encapsulating material, and dispersed throughout the chewing gum composition. In a most preferred embodiment, the composition includes carbamide peroxide or urea peroxide. Even further, peroxides have the added benefit of providing a breath freshening effect to the user, in addition to the whitening effect provided. Even further still, when peroxides react with water, such as saliva, they have a tendency to provide a foaming action, which not only adds to the whitening ability of the product, but also provides the user with a mouth feel, or sensorial cue, that can be associated with the product "working". As explained above, however, peroxides are typically not used in chewing gum products due to their high tendency to react with the gum base, causing degradation, and thus reducing their effectiveness and their shelf life. The present inventors have discovered that certain encapsulation materials in certain amounts can provide a very useful tooth whitening effect, even when used in chewing gum.

It is particularly desirable that the solid peroxide composition be completely encapsulated, since even a slight exposure to water or water-based materials may react with the peroxide and render it ineffective. For example, without encapsulation of carbamide peroxide in a chewing gum product, after two weeks of storage nearly all of the carbamide peroxide is decomposed in the product. However, if the carbamide peroxide is encapsulated, a majority of the initial peroxide is still present and effective after at least 8-12 weeks. Desirably, at least 70% of the initial peroxide is still present and effective in the chewing gum product after about 10 weeks. The preferred encapsulation material will be water-insoluble, and free of any water-based components. Further, the preferred encapsulation material should be capable of providing a water-tight film around the peroxide that is strong enough to withstand the rigorous processing associated with chewing gum products, but that also will break apart when the product is chewed by the user. Further, it is desirable that more peroxide material be incorporated into the product, and thus there is desirably more peroxide material than encapsulating material present in each of the active-containing particles.

In a preferred embodiment, the tensile strength of the encapsulation material ranges from about 4000 to about 300,000 psi after the heating step, including 5000, 10000, 25000, 50,000, 75000, 90,000, 100000, 125000, 155000, 180000, 205000, 230000, 255000, 270000, 295000 psi and all values and subranges there between. A dual encapsulation system is used. In the dual encapsulation, the encapsulated peroxide includes three regions: the inner region including the solid peroxide composition; a middle region including a first encapsulating material; and an outer region including a second encapsulating material. The first encapsulating material comprises polyvinyl acetate, and the second encapsulating material comprises a cellulosic material, such as ethyl cellulose.

The encapsulated peroxide composition may be in the form of a plurality of individually encapsulated particles. The particles may have an average particle size of from 50 to 1500 microns in diameter, more particularly 250 to 750 microns in
diameter, and even more desirably have an average particle size of 500 microns in diameter.

There is desirably a higher amount of peroxide composition than encapsulating material in each particle. Thus, the ratio of peroxide to encapsulating material is desirably greater than 1:1, and most desirably is from 1:1 to 4:1. Even further, it is most desirable that the level of peroxide be as high as possible, while still maintaining a suitable encapsulation effect. If more peroxide can be incorporated into the composition, a greater stain removing effect may be seen. In some embodiments, the encapsulated peroxide includes 20% to 50%
by weight encapsulating material(s) and 80% to 50% by weight peroxide. In some embodiments, the encapsulated peroxide includes 20% to 30% encapsulating material(s), and in most preferred embodiments, the encapsulated peroxide includes 20% to 25% encapsulating material(s).

Any desirable encapsulating methods may be used to prepare the encapsulated peroxide particles, including, for example, spray coating, hot melt extrusion, spray chilling and spinning disk methods. Water insoluble encapsulating materials, such as ethyl cellulose, are especially preferred in the formation of the encapsulated particles, due to their processing ability. Certain water soluble materials have a tendency to become very sticky and highly viscous during processing, which renders the complete coating of the peroxide material difficult.

When using polyvinyl acetate, it may be preferred to use an extrusion method, whereby the solid carbamide peroxide and the polymeric compound are first mixed together. Since carbamide peroxide has a tendency to degrade at temperatures at or above 70 °C, it is beneficial to limit exposure to heat during the extrusion process to below 70°C. Preferably, the combination of peroxide and polymeric matrix are heated to a temperature of 65°C or less, and then extruded. For example, in one method, the polymeric material (e.g., polyvinyl acetate) is melted at a temperature of about of 65°C in a high shear mixer such as extruder (single or twin screw) or sigma or Banbury mixer. Other additional encapsulating materials may be added and mixed if desired. The desired encapsulated material (e.g., carbamide peroxide) is added to the melted polymeric material and mixed under high shear to completely disperse the ingredients, and the resulting mixture is extruded. The resulting filled polymer melt is cooled and ground to the desired particle size. If the encapsulated material is not directly added to the chewing gum product, it should be stored in a suitable container, most desirably an air tight container at a controlled temperature.

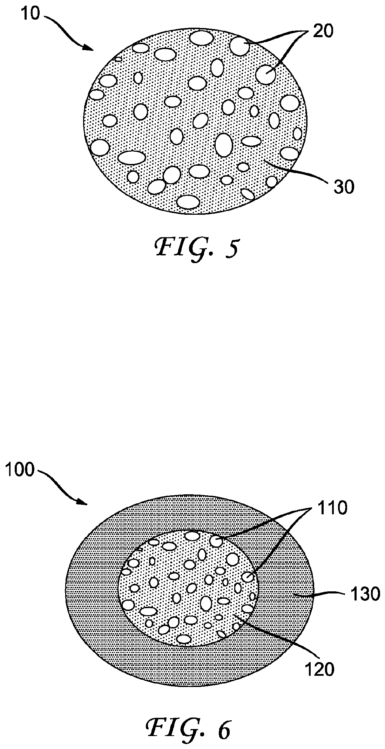

The cooled extruded material is ground into particles of the desired size. Sizes may include from 200-700 microns in diameter, and more specifically 200-400 microns in diameter. In such embodiments, it is desired to have at least 60% by weight of the encapsulated material be the polymeric matrix, so as to provide sufficient binding and encapsulation of the peroxide. Most desirably, the encapsulated material is 10-40% by weight peroxide, and even more desirably 30% by weight peroxide. In the dual encapsulated system, the resulting ground particles are then be coated with a cellulosic material, such as ethyl cellulose, via any desired methods. Benefits of using a polyvinyl acetate encapsulation material include the tendency of polyvinyl acetate to provide a strong crystalline structure, protecting the peroxide housed therein. As can be seen in Figure 5, oneembodiment of a polymer-CP encapsulation matrix 10 is seen, with the CP particles shown as 20 and the polymeric matrix shown as 30.

The peroxide may be encapsulated at any temperature, and in some embodiments is encapsulated in an outside temperature of 25-65°C, giving a product temperature of 25-50°C. It may be desirable to dissolve the encapsulating material in a solvent before coating the peroxide. For example, the encapsulating material may be dissolved in a solution of ethanol, ammonium hydroxide, or iso-propanol acetone/ethanol (80/20). The encapsulating material may be dissolved in the solvent in any desired amount, such as from 10% to 25% by weight encapsulating material. In one preferred embodiment, for example, ethyl cellulose may be dissolved in the acetone/ethanol (80/20) solvent in an amount of 12% encapsulating material. The resulting solution may be used to coat the particles of peroxide material. In the case of dual coated particles, the outer encapsulating material may be dissolved in water or a water-based solvent prior to coating the coated particle. Spray coating is preferred, however, it may alternatively be desired that the solid peroxide particles be dispersed in a molten fat or wax composition, and solidified using a spinning disk encapsulation method or a spray chilling encapsulation method.

The encapsulated product may include from about 5% to about 20% first encapsulating material in the middle region and from about 10% to about 30% second encapsulating material in the outer region.

Figure 6 shows a schematic diagram of one embodiment of a double encapsulation system 100, where the CP particles are seen as 110, the encapsulating polymer is seen as 120, and the ethyl cellulose covering is seen as 130.

The products of the present invention include a plurality of encapsulated peroxide particles dispersed throughout the product. In the case of a chewing gum product, the chewing gum includes a plurality of encapsulated peroxide particles dispersed throughout the gum base. Desirably, the products of the present invention include a peroxide composition in an amount of from 0.1% to 10% by weight of the chewing gum product, and more desirably from 1% to 6% by weight of the chewing gum product, and most desirably from 2% to 4% by weight of the chewing gum product. In some embodiments, the product may include 3% solid peroxide composition by weight of the chewing gum product, while in other embodiments, the product may include 6% solid peroxide composition by weight of the chewing gum product. For encapsulated peroxide particles, the amount of the encapsulated peroxide particle may be present in the chewing gum in an amount of from 1 % to 20% by weight of the chewing gum product, depending upon the amount of encapsulating material used in the particles. It is particularly desirable that there be sufficient peroxide composition to allow for a tooth whitening effect, but still provide a favorable taste to the user.

The use of a suitable encapsulating material is particularly important for the inventive peroxide-containing products, as the encapsulation will provide a suitably long shelf life. In some embodiments, it is desirable that the product have a shelf life of at least three months under normal storage conditions and at approximately room temperature, and in other embodiments the shelf life is desirably at least six months. Most desirably, the product has a shelf life of at least one year. As used herein, the term "shelf life" means that the finished product remains useful and effective after a particular amount of time. It is, of course, understood that some of the peroxide composition in the finished product may undesirably be reacted and rendered ineffective, but the present invention maintains that amount of ineffective peroxide as low as possible. Desirably, a useful shelf life will have at least 70% of the peroxide effective after the desired time, and more desirably at least 80% of the peroxide will be effective after the desired time.

The chewing gum products of the present invention may include a sweetener, where the sweetener is desirably non-reactive with the peroxide composition. It has been found that sweeteners such as aspartame and glycerin have a tendency to react with the peroxide, while sweeteners such as sucralose, isomalt and maltitol do not react with the peroxide. For this reason, sucralose, isomalt and maltitol, and combinations thereof, are preferred in the product. Of course, other sweeteners may be used, if desired.

Carbamide peroxides are known to react in the presence of water, such as saliva, which acts to break down the carbamide peroxide into hydrogen peroxide, which then dissociates, creating a free peroxide radical. This free peroxide radical performs the desired stain removing effect. Although the presence of saliva aids in breaking down the product and allowing the desired effect, products of the present invention may include one or more activators to speed up the process and aid in whitening. Suitable activators include transition metals, such as iron or manganese. Due to the catalytic effect of the activator, it is particularly desired that the activator and the peroxide be maintained in separate regions of the product during processing and storage. For example, the peroxide may be encapsulated and the activator may be free, and both may be dispersed in a chewing gum base. The presence of the encapsulating material will keep the two from prematurely reacting. During use, when the user chews the product and breaks apart the encapsulant shell, the activator and the peroxide are allowed to react, resulting in a useful whitening composition.

The products of the present invention may further include a polyphosphate, and in desired embodiments include sodium tripolyphosphate. Additional tooth whitening agents, such as polyphosphates, may work in conjunction with the peroxide to increase the tooth whitening effect of the inventive product. Polyphosphates may be encapsulated or they may be freely dispersed throughout the product. The polyphosphate may be present in an amount of from 1% to 5% by weight of the product, and more particularly 4% by weight of the product. Specifically when the product includes 4% by weight of the peroxide, it may be useful to use 4% by weight of a polyphosphate, such as sodium
tripolyphosphate. Inclusion of a polyphosphate may add benefits as increased future stain prevention, as well as stain weakening for easier removal.

In some embodiments, the products may additionally include a casein phosphopeptide-calcium phosphate complex, commonly referred to as CPP-ACP, or casein phosphopeptide-calcium fluorophosphates, commonly referred to as CPP-ACFP. CPP-ACP and CPP-ACFP provide remineralization to teeth surfaces, but also provides teeth with reduced sensitivity. In some instances, exposure of teeth to peroxides may result in the user's teeth experiencing sensitivity. CPP-ACP or CPP-ACFP may reduce the sensitivity levels experienced by the user. If used, CPP-ACP or CPP-ACFP may be used in amounts from 0.1% to 5% by weight of the composition.

There is also provided a method of whitening teeth in a user. In this method, an orally acceptable product is provided, which includes a carrier and a plurality of encapsulated peroxide particles. In preferred embodiments the carrier is a gum base and the peroxide is a solid peroxide composition, including carbamide peroxide. The encapsulating material is as described above.

The amounts of the relative products are described in further detail above.

Desirably, the peroxide is released from the carrier, i.e., a gum base, within less than 30 seconds, and more desirably within less than 20 seconds. It is further desirable that substantially all of the peroxide be released from the product within 5-10 minutes of mastication. In view of the quick release of peroxide, and the noticeable taste to the user, it may be desirable to incorporate a free flavor and/or sweetener in the product to provide taste masking to the user. Further, the peroxide will remain in the oral cavity of the user for an extended period of time, providing the stain removing effect to teeth. For this reason, it may be desirable to include at least one encapsulated sweetener and/or flavor, to provide extended taste masking to the user.

The product is provided to the user, who then proceeds to chew the product. The act of chewing the product effectively breaks the encapsulant barrier, freeing the peroxide. The peroxide is then allowed to react in the presence of saliva and optionally one or more activators, thus providing a tooth whitening effect.

In addition, the activated peroxide has a tendency to form a foam, which provides an increased amount of contact with the tooth surface, aiding in the tooth whitening effect. As an additional feature, the presence of the foaming action is a signal to the user that the product is working effectively, which provides the user with a satisfactory feeling that the product is acting as desired. Even further, peroxides have been known to provide a breath freshening effect to the user, in addition to the whitening effect. Thus, in some embodiments, the method includes not only whitening of teeth, but also providing a breath freshening effect. The products may include a solid peroxide composition in an amount of about 1-2% by weight of the product to provide a suitable breath freshening effect.

The product is useful in providing tooth whitening to the user. Whitening of teeth is analyzed by comparing the initial level of stain on the teeth to the level of stain on the teeth after use.. Stains and stain-removing effect may be measured in any desired manner, and in some embodiments the stain removing effect may be determined using the CIELABequation: ΔE= [(AL*)² + (Δa*)² + (Δb*)²]^{½}, In this equation, L* represents whiteness, a* represents red-green color and b* represents yellow-blue color. The color values for each of the three measurements may be taken using diffuse reflectance absorbance readings with a spectrophotometer. In some embodiments, an average of multiple readings may be used to provide the value. Readings for each color category may be taken before any stain removing product is applied to the teeth, and then differences may be calculated by measuring the stain levels after use. The ΔE value summarizes the overall change for each color factor and represents the ability of the test product to remove stain and whiten teeth. The human eye can typically detect a ΔE color change of 2 or larger, and thus it is desirable that the product provided herein provide a stain removing effect of at least 2 as measured on the CIELAB scale. The higher the number for ΔE, the greater the stain removing effect,

### Examples

### Example 1- Stability of Encapsulated Carbamide Peroxide

The stability of various encapsulants for carbamide peroxide in chewing gum was measured. As explained above, peroxides in general are known to be problematic in the shelf life of chewing gums, as they have a tendency to break down gum base, rendering it ineffective and useless. Chewing gums including various encapsulated carbamide peroxides were prepared and were stored for eight weeks at room temperature and at 37°C. Solid carbamide peroxide was encapsulated in the following encapsulating materials: (1) ethyl cellulose (25% encapsulant weight); (2) polyvinyl acetate B30 (30% encapsulant weight); (3) gum arabic (30% encapsulant weight); (4) mannitol (30% encapsulant weight); and (5) shellac (30% encapsulant weight). These encapsulated particles were used in formulating pieces of chewing gum and were measured for shelf life stability. In addition, a control chewing gum was prepared, which included non-encapsulated ("free") carbamide peroxide within the gum base. The amount of carbamide peroxide obtained after the study was measured.

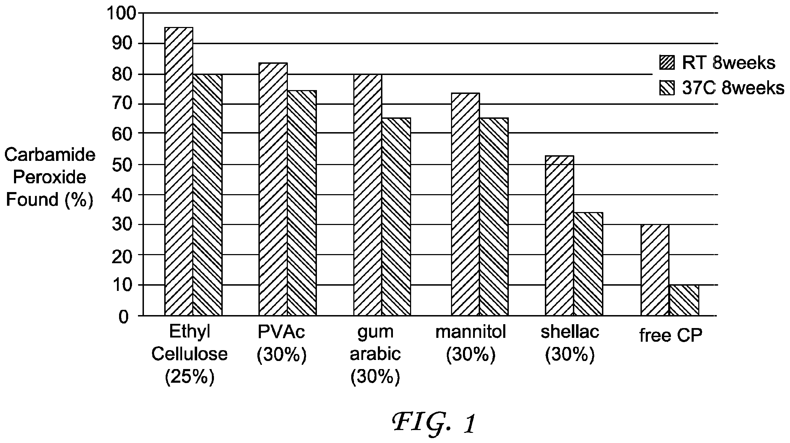

The results are provided in Figure 1. As can be seen, significant results were obtained through the use of a low encapsulant weight of ethyl cellulose (25% by weight of the encapsulated particle). At room temperature after eight weeks, chewing gums including this encapsulated carbamide peroxide had approximately 95% of the carbamide peroxide remaining. Even after eight weeks at elevated temperatures, these products had approximately 80% of the carbamide peroxide remaining. Polyvinyl acetate, gum arabic and mannitol provided beneficial results, as well. It was found that using shellac as an encapsulant did not provide adequate stability, and free, unencapsulated carbamide peroxide had the lowest stability.

### Example 2 - Determination of Optimal Coating Weight

Having determined that ethyl cellulose provides a significant stability level to the carbamide peroxide, the determination of the optimal encapsulating weight of ethyl cellulose was evaluated. The goal was to provide a secure encapsulation of the peroxide, so that the peroxide would remain encapsulated during processing of the chewing gum composition, while still maintaining high levels of peroxide to be effective.

Ethyl cellulose was coated onto carbamide peroxide in coating levels of 5% ethyl cellulose, 10% ethyl cellulose, 20% ethyl cellulose, 25% ethyl cellulose, 30% ethyl cellulose, and 50% ethyl cellulose (all by weight of the encapsulated particle). A 20 gram sample of each of the encapsulated particles was prepared and introduced into a half liter container of water. The water was then swirled for about 5 minutes, filtered and dried overnight. The remaining materials were weighed and compared to the initial weight.

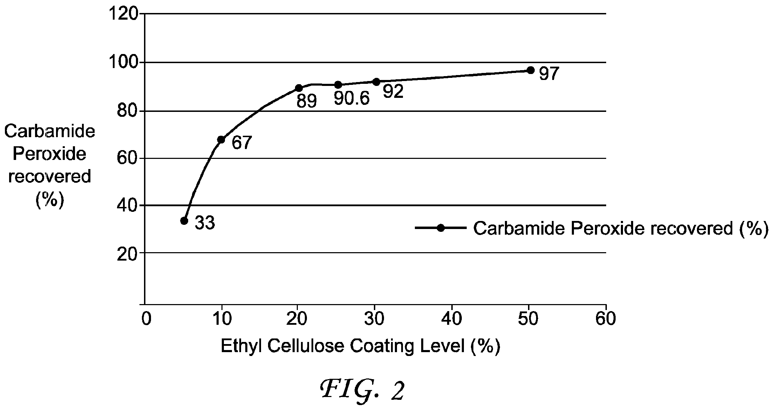

As can be seen in Figure 2, the low coating weight of ethyl cellulose (5%, 10%) had very low stability. The recovery of materials at 5% encapsulation weight was 30%, while the recovery of materials at 10% encapsulation weight was 51%. This was presumably due to the fact that the amount of ethyl cellulose used to coat the carbamide peroxide was insufficient to fully coat the peroxide, allowing water to react with the peroxide. Given peroxide's highly reactive nature with water, even the slightest contact with water will react, reducing its stability.

It was surprising that at a 20% encapsulant weight (i.e., 20% ethyl cellulose, 80% carbamide peroxide), the recovery level was 91%. The recovery level only marginally increased above 20% encapsulation weight (25% encapsulation weight provided 92% recovery; 30% encapsulation weight provided 96% recovery; and 50% encapsulation weight provided 99% recovery). Thus, it can be determined that using 20%-50% encapsulation weight provides significant stability for the peroxide, and in fact, using 20%-25% encapsulation weight provides nearly as significant stability for the peroxide.

This example demonstrates that an effective encapsulation weight for ethyl cellulose is from about 20% to about 50% encapsulant; and may include from about 20% to about 30% encapsulant; and may include from about 20% to about 25% encapsulant. Having a lower encapsulant weight allows for a higher amount of peroxide to be used in the chewing gum composition without sacrificing stability.

### Example 3 - In Vitro Whitening Studies

Samples of whitening gum were prepared, along with control samples of a chewing gum (including no peroxide composition). The encapsulated peroxide particles used in the inventive tooth whitening chewing gum had a particle size of from 100-900 microns, with an average particle size of about 500 microns in diameter. The encapsulated peroxide particles included a 25% ethyl cellulose outer coating and 75% peroxide inner core. Each piece was approximately 1 gram in weight. The chewing gums were used in two separate studies. The first study was an initial study testing the whitening effect of the gums over a shortened period, where twelve pieces of each gum were chewed, two pieces at a time for twenty minutes during each chew period. The second study was conducted over a longer period of time, using the first three gums. This second study used an artificial mouth containing bovine teeth, and the whitening effect was measured over 7 days and 14 days. The five gum compositions used in the studies were prepared as slab gum pieces, and included the following components as set forth in Table 2 below:

**Table 2: Chewing Gum Compositions**

| **Components** | **Gum 1 (control) (%)** | **Gum 2 (3% CP) (%)** | **Gum 3 (6% CP) (%)** | **Gum 4 (6% CP) (%)** | **Gum 5 (2% CP) (%)** |
|---|---|---|---|---|---|
| Gum Base | 28 | 28 | 28 | 28 | 28 |
| Lecithin | 0.5 | 0.5 | 0.5 | 0.5 | 0.5 |
| Sorbitol | QS | QS | QS | QS | QS |
| Glycerin | 5 | 5 | 5 | 5 | 5 |
| Sucralose | 1 | 1 | 1 | 1 | 1 |
| Flavors | 2.5 | 2.5 | 2.5 | 2.5 | 2.5 |
| Taste masking agent | 0.35 | 0.35 | 0.35 | 0.35 | 0.35 |
| Flavor Enhancer | 0.5 | 0.5 | 0.5 | 0.5 | 0.5 |
| Encapsulated Carbamide peroxide | 0 | 3 | 6 | 6 | 2 |
| Sodium tripolyphosphate | 0 | 0 | 0 | 4 | 2 |
| Manganese Gluconate | 0 | 0 | 0 | 0 | 0.6 |

### Study 1 - Shortened Study

In the first study, a total of twelve pieces of each of the five chewing gums from Table 2 were analyzed. The chewing gums were used in an artificial chewing apparatus, including a pump and flow cell into which 12 stained bovine teeth were placed. For each chew period, two chewing gum pieces were masticated for 20 minutes at a time until all twelve pieces were masticated. After each mastication period, the peroxide that was released into the artificial saliva was circulated through the flow cell, contacting the stained bovine teeth. The whitening efficacy of the composition on the teeth was evaluated by color change, which was measured by using the CIELAB equation described above, taking the values before use and after the twelve pieces of gum were chewed.

The ΔE values were measured after all twelve pieces were chewed. The results are set forth in Table 3 below:

**Table 3: Results of chewing Twelve Pieces of Gum**

| **Gum Sample** | **ΔE** |
|---|---|
| **Gum 1** | 0.4 |
| **Gum 2** | 1.0 |
| **Gum 3** | 2.2 |
| **Gum 4** | 1.3 |
| **Gum 5** | 1.8 |

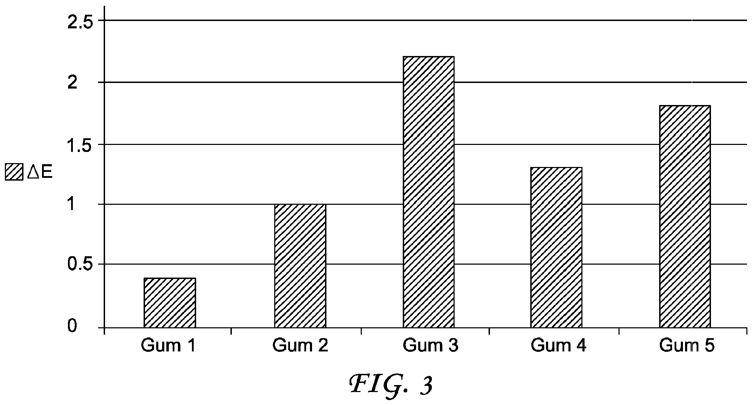

The results of the first study are depicted in Figure 3. As can be seen, control gum **1** had negligible whitening effect, while gums 2-5 each had at least a one-fold increase in whitening. Chewing gum 3 produced the most noticeable whitening effect over the shortened period.

### Study 2 - Seven-Day and Fourteen-Day Study

In the second study, gums 1-3 from Table 2 above were evaluated over a longer evaluation period. The longer evaluation period included analysis at seven days and at fourteen days. Again, the chewing gums were used in an artificial chewing apparatus, including a pump and flow cell into which 12 stained bovine teeth were placed. Each chewing gum piece was masticated for 20 minutes at a time, four times per day, for a period of 7 days and a period of 14 days. After each mastication period, the peroxide that was released into the artificial saliva was circulated through the flow cell, contacting the stained bovine teeth. The whitening efficacy of the composition on the teeth was evaluated by color change, which was measured by using the CIELAB equation described above, taking the values before use, after one week of continued use, and after two weeks of continued use. The ΔE values after one week and after two weeks were measured.

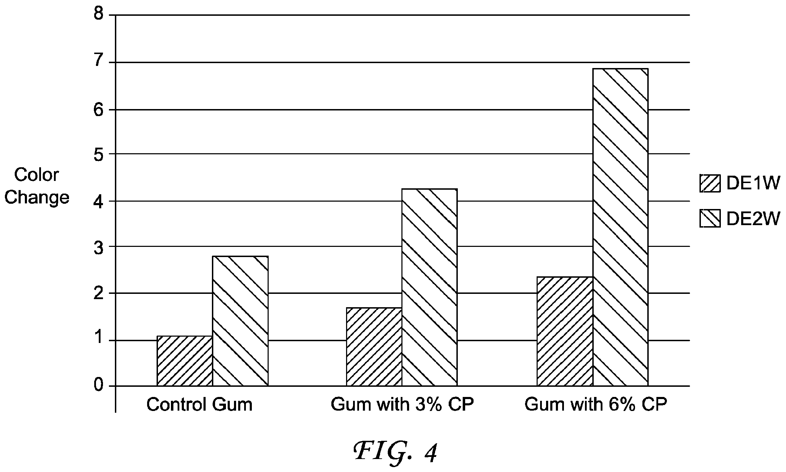

The results are set forth in Figure 4. As can be seen, there is a marked improvement in the whitening effect when the peroxide is used in either 3% by weight of the composition or 6% by weight of the composition. After 1 week of chewing, the control gum provided slight whitening effect (color change of 1 degree), which would not be detectable by human eyesight. The 3% peroxide gum provided about 1.8 degree color change, which is slightly visible by human eyesight, and the 6% peroxide gum provided about 2.25 degree color change. However, over a two-week period, the disparity in results is even more demonstrated. The control gum provided about a 2.8 degree color change, just above that which is visible by human eyesight, while the 3% peroxide gum provided 4.1 degree change and the 6% peroxide chewing gum provided nearly a 7 degree color change.

Over a two-week period, the chewing gum containing 3% carbamide peroxide provided an improved whitening effect of about 1.46 x the level of the control gum. Over the same two-week period, the chewing gum containing 6% carbamide peroxide provided an improved whitening effect of nearly 2.5 x the level provided by the control gum. Both the 4.1 degree change and the 7 degree change are significant and provide clearly visible results with normal human eyesight. The results show clearly that the peroxide-containing gum provides a marked improvement in the whitening effect over a control chewing gum, especially over a 14-day period.

### Example 4 - Matrix Encapsulation

Various samples of encapsulated peroxide were made via hot melt extrusion. A first batch was made including 350g of PVAc (B17) and 150g of Carbamide peroxide. The PVAc and carbamide peroxide were premixed and extruded using a twin screw extruder at 65 °C. The extrudate was stored at room temperature for 24 hrs before milling to a desired particle sizes. The particle size distribution was about 200 to about 700 microns.

A second batch was made having a double encapsulation system. PVAc particles containing carbamide peroxide 30% w/w were prepared as explained above and then subsequently coated with ethyl cellulose using a spray coating process (solvent evaporation) to create a core-shell 80:20 w/w encapsulation system. Ethyl cellulose was dissolved in Iso-propanol-Acetone solvent system.

As set forth in Tables 4A and 4B below, four chewing gum compositions (A-D) were prepared including various amounts of components, and their stabilities were evaluated. The four chewing gum compositions (A-D) included different amounts and types of components, including free (unencapsulated) carbamide peroxide.

**Table 4A - Compositions A and B**

| **Ingredient** | **Composition A%** | **Composition B%** |
|---|---|---|
| Gum Base | 25 | 25 |
| Maltitol | 35 | 35 |
| Sorbitol | QS | QS |
| Carbamide Peroxide - PVAc System | 8 | 0 |
| Carbamide eroxide-PVAc- Ethyl Cellulose System | 0 | 13 |
| Carbamide Peroxide- Ethyl Cellulose System | 0 | 0 |
| Carbamide Peroxide | 0 | 0 |
| Flavor | 2.5 | 2.5 |
| Sucralose | 1.2 | 1.2 |
| Glycerin | 2 | 2 |
| **Total** | **100** | **100** |

**Table 4B - Compositions C and D**

| **Ingredient** | **Composition C %** | **Composition D %** |
|---|---|---|
| Gum Base | 25 | 25 |
| Maltitol | 35 QS | 35 |
| | Sorbitol | QS |
| Carbamide Peroxide - PVAc System | 0 | 0 |
| Carbamide peroxide- PVAc- Ethyl Cellulose System | 0 | 0 |
| Carbamide Peroxide- Ethyl Cellulose System ystem | 4.8 | 0 |
| Carbamide Peroxide | 0 | 4 |
| Flavor | 2.5 | 2.5 |
| Sucralose | 1.2 | 1.2 |
| Glycerin | 2 | 2 |
| **Total** | **100** | **100** |

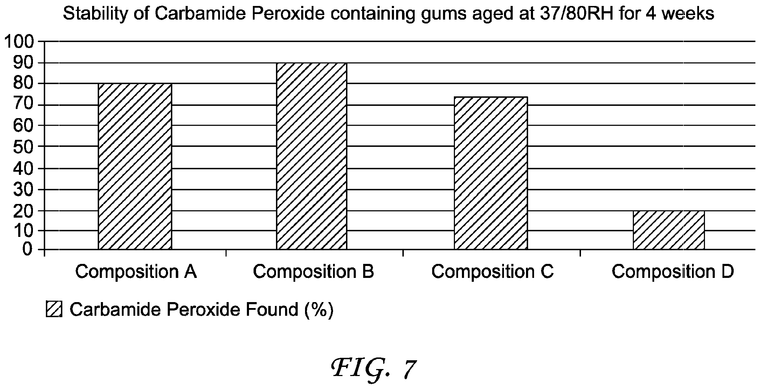

The four chewing gum products were then evaluated for stability by aging them at 30 °C and 80% RH for a period of four weeks. The results are set forth in Figure 7. As can be seen, Composition D, which included unencapsulated (free) carbamide peroxide, demonstrated less than 20% stability after the aging period. This demonstrates an exceptionally poor product. Compositions A, B and C all demonstrated increase in stability, with each showing more than 70% carbamide peroxide found after the aging process. Composition B demonstrated the highest stability, with 90% carbamide peroxide found after the process was complete, while Composition A still showed greater than 80% stability.

### Example 5 - Clinical study results

Four experimental chewing gum prototypes were prepared and evaluated for the ability to remove extrinsic tooth stain. The four chewing gum prototypes were compared to a control product: Trident White, which includes 0.5% sodium stearate as its active ingredient. The study was a double-blind parallel, controlled clinical study, in which 243 individuals were instructed to chew the product four times per day (which included once after each meal and once before going to bed), for twenty minutes per chew period. The stain level of the teeth of the individuals was evaluated at the baseline (0 days), after three weeks, after eight weeks, and after twelve weeks. Results were measured using the Vitapan Classic Shade Guide and also the MacPherson Modification of the Lobene Stain Index (MMLS).

Sample A was a pellet gum including 4% by weight carbamide peroxide and 2% sodium tripolyphosphate. Sample B was a pellet gum including 0.5% sodium stearate and 2% sodium tripolyphosphate. Sample C was the control sample, including a pellet gum having 0.5% sodium stearate. Sample D was a pellet gum including 2% sodium tripolyphosphate, and Sample E was a pellet gum including 4% sodium tripolyphosphate.

The results of the study are set forth in Table 5 below, as measured by the Vitapan Guide, where the values reflect the change from baseline:

**Table 5 - results of clinical study**

| | **3 wks** | **8 wks** | **12 wks** |
|---|---|---|---|
| 4% Carbamide Peroxide and 4% STP | 0.2 | 1.2 | 2.1 |
| 0.5% Sodium Stearate & 2% STP | 0.1 | 0.8 | 1.6 |
| current Trident White | 0.0 | 0.6 | 1.3 |
| 2% STP | 0.1 | 0.8 | 1.6 |
| 4% STP | 0.1 | 1.0 | 1.9 |

As can be seen, although all samples produced a stain removal effect on the teeth of the user, the only composition that reached a two-fold shade increase was the combination of using 4% carbamide peroxide and 4% sodium tripolyphosphate. Reaching a two-fold whitening increase is important as it allows the manufacturer of a product to legally and justifiably claim that it results in two shades whiter teeth to the user.

## Claims

1. A tooth whitening chewing gum composition comprising:
(a) a chewing gum base; and
(b) a plurality of active-containing particles, each of said active-containing particles comprising an inner region comprising a solid peroxide composition, a middle region comprising a first encapsulating material, and an outer region comprising a second encapsulating material,
wherein the first encapsulating material comprises polyvinyl acetate and the second encapsulating material comprises a cellulosic material; and
each of said active-containing particles comprises between 20-50% by weight encapsulating material and between 50-80% by weight of peroxide.

2. A method of whitening the teeth of a user, comprising the steps of:
(a) preparing a tooth whitening chewing gum composition comprising:
(i) a chewing gum base; and
(ii) a plurality of active-containing particles, each of said active-containing particles comprising an inner region comprising a solid peroxide composition, a middle region comprising a first encapsulating material, and an outer region comprising a second encapsulating material comprising a cellulosic material, wherein the first encapsulating material comprises polyvinyl acetate and the second encapsulating material comprises a cellulosic material; and wherein each of said active-containing particles comprises between 20-50% by weight of encapsulating material and between 50-80% by weight of peroxide;
(b) providing said chewing gum composition to a user; and
(c) said user chewing said chewing gum composition, wherein said chewing breaks said outer region and release said inner region into the mouth, thereby exposing said user's teeth to said peroxide composition.

3. The gum composition of claim 1 or the method of claim 2, wherein said solid peroxide composition is selected from the group consisting of: carbamide peroxide, sodium percarbonate, peroxydone, calcium peroxide, perbenzoic acid, and combinations thereof.

4. The gum composition of claim 1 or the method of claim 2, wherein said outer region comprises a layer of at least one encapsulating material selected from the group consisting of ethyl cellulose, polyvinyl acetate, shellac, wax, fat, lipid, emulsifier, gum arabic, mannitol, HPMC, other cellulose derivatives, polyethylene glycol, gelatin and combinations thereof.

5. The gum composition of claim 1, wherein each of said active-containing particles comprises between 10- 50% of said outer region by weight of said particle and between 50-90% of said inner region by weight of said particle.

6. The gum composition of claim 1, wherein said chewing gum composition comprises 0.5% to 6% of said solid peroxide composition by weight of said gum composition.

7. The gum composition of claim 1, wherein said chewing gum composition comprises 0.5% to 25% of said encapsulated peroxide particle by weight of said gum composition.

8. The gum composition of claim 1 or the method of claim 2, wherein each of said active-containing particles comprises at least two separate encapsulating materials.

9. The gum composition or method of claim 8, wherein each of said active-containing particles comprises said inner region comprising a solid peroxide composition, a first outer region surrounding said inner region, and a second outer region surrounding said first outer region, wherein said first and second outer regions comprise different encapsulating materials.

10. The gum composition or method of claim 9, wherein said first outer region comprises a hydrophobic material and said second outer region comprises a hydrophilic material.

11. The method of claim 2, wherein each of said active containing particles comprises between 20-50% of said outer region by weight of said particle and between 50- 80% of said inner region by weight of said particle.

12. The method of claim 2, wherein said chewing gum composition comprises 0.5% to 10% of said solid peroxide composition by weight of said gum composition.

13. The method of claim 2, wherein said chewing gum composition comprises 1% to 20% of said encapsulated peroxide composition by weight of said gum composition.

14. The method of claim 2, wherein said gum composition further comprises a chelating agent selected from the group consisting of 0.5-6% polyphosphates, pyrophosphates, sodium tripolyphosphate, polyphosphonates and combinations thereof, by weight of the gum composition.

15. A tooth whitening chewing gum composition as claimed in claim 1 comprising:
(a) a chewing gum base; and
(b) a plurality of active-containing particles, each of said active-containing particles comprising a solid peroxide composition within a polymeric encapsulation matrix.

16. The composition of claim 15, wherein said active-containing particle further comprises a coating of ethyl cellulose.

17. A method of whitening the teeth of a user as claimed in claim 2, comprising the steps of:
(a) preparing a tooth whitening chewing gum composition comprising the steps of:
(i) providing a chewing gum base;
(ii) separately providing a plurality of active-containing particles prepared by the steps of:
(1) mixing a polymeric material and a solid peroxide composition to provide a mixture;
(2) extruding said mixture;
(3) cooling said mixture; and
(4) grinding said cooled mixture to provide a plurality of active-containing particles;
(iii) combining said chewing gum base and said plurality of active-containing particles to provide a resulting chewing gum composition;
(b) providing said chewing gum composition to a user; and
(c) said user chewing said chewing gum composition, wherein said chewing releases said solid peroxide composition, thereby exposing said user's teeth to said peroxide composition.

## Patentansprüche

1. Zahnweißkaugummizusammensetzung, die umfasst:
(a) eine Kaugummibasis; und
(b) eine Vielzahl aktivstoffhaltiger Partikel, wobei jedes der aktivstoffhaltigen Partikel ein Innengebiet, das eine feste Peroxidzusammensetzung umfasst, ein mittleres Gebiet, das ein erstes Kapselungsmaterial umfasst, und ein Außengebiet, das ein zweites Kapselungsmaterial umfasst, umfasst,
wobei das erste Kapselungsmaterial Polyvinylacetat umfasst und das zweite Kapselungsmaterial ein Zellulosematerial umfasst; und
jedes der aktivstoffhaltigen Partikel zwischen 20-50 Gew.-% Kapselungsmaterial und zwischen 50-80 Gew.-% Peroxid umfasst.

2. Verfahren zum Weißen der Zähne eines Nutzers, wobei das Verfahren die folgenden Schritte umfasst:
(a) Zubereiten einer Zahnweißkaugummizusammensetzung, die umfasst:
(i) eine Kaugummibasis; und
(ii) eine Vielzahl aktivstoffhaltiger Partikel, wobei jedes der aktivstoffhaltigen Partikel ein Innengebiet, das eine feste Peroxidzusammensetzung umfasst, ein mittleres Gebiet, das ein erstes Kapselungsmaterial umfasst, und ein Außengebiet, das ein zweites Kapselungsmaterial umfasst, das ein Zellulosematerial umfasst, umfasst, wobei das erste Kapselungsmaterial Polyvinylacetat umfasst und das zweite Kapselungsmaterial ein Zellulosematerial umfasst; und wobei jedes der aktivstoffhaltigen Partikel zwischen 20-50 Gew.-% Kapselungsmaterial und zwischen 50-80 Gew.-% Peroxid umfasst;
(b) Bereitstellen der Kaugummizusammensetzung für einen Nutzer; und
(c) wobei der Nutzer die Kaugummizusammensetzung kaut, wobei das Kauen das Außengebiet aufbricht und das Innengebiet in dem Mund freisetzt und dadurch die Zähne des Nutzers der Peroxidzusammensetzung aussetzt.

3. Gummizusammensetzung nach Anspruch **1** oder Verfahren nach Anspruch 2, wobei die feste Peroxidzusammensetzung aus der Gruppe ausgewählt ist, die besteht aus: Carbamidperoxid, Natriumpercarbonat, Peroxydone, Calciumperoxid, Peroxybenzoesäure und Kombinationen davon.

4. Gummizusammensetzung nach Anspruch **1** oder Verfahren nach Anspruch 2, wobei das Außengebiet eine Schicht wenigstens eines Kapselungsmaterials umfasst, das aus der Gruppe ausgewählt ist, die aus Ethylzellulose, Polyvinylacetat, Schellack, Wachs, Fett, Lipid, Emulgator, Gummiarabikum, Mannit, HPMC, Polyethylenglycol, Gelatine und Kombinationen davon besteht.

5. Gummizusammensetzung nach Anspruch 1, wobei jedes der aktivstoffhaltigen Partikel zwischen 10-50 % des Außengebiets, bezogen auf das Gewicht des Partikels, und zwischen 50-90 % des Innengebiets, bezogen auf das Gewicht des Partikels, umfasst.

6. Gummizusammensetzung nach Anspruch 1, wobei die Kaugummizusammensetzung 0,5 % bis 6 % der festen Peroxidzusammensetzung, bezogen auf das Gewicht der Gummizusammensetzung, umfasst.

7. Gummizusammensetzung nach Anspruch 1, wobei die Kaugummizusammensetzung 0,5 % bis 25 % des gekapselten Peroxidpartikels, bezogen auf das Gewicht der Gummizusammensetzung, umfasst.

8. Gummizusammensetzung nach Anspruch **1** oder Verfahren nach Anspruch 2, wobei jedes der aktivstoffhaltigen Partikel wenigstens zwei getrennte Kapselungsmaterialien umfasst.

9. Gummizusammensetzung oder Verfahren nach Anspruch 8, wobei jedes der aktivstoffhaltigen Partikel das Innengebiet, das eine feste Peroxidzusammensetzung umfasst, ein erstes Außengebiet, das das Innengebiet umgibt, und ein zweites Außengebiet, das das erste Außengebiet umgibt, umfasst, wobei das erste und das zweite Außengebiet unterschiedliche Kapselungsmaterialien umfassen.

10. Gummizusammensetzung oder Verfahren nach Anspruch 9, wobei das erste Außengebiet ein hydrophobes Material umfasst und wobei das zweite Außengebiet ein hydrophiles Material umfasst.

11. Verfahren nach Anspruch 2, wobei jedes der aktivstoffhaltigen Partikel zwischen 20-50 % des Außengebiets, bezogen auf das Gewicht des Partikels, und zwischen 50-80 % des Innengebiets, bezogen auf das Gewicht des Partikels, umfasst.

12. Verfahren nach Anspruch 2, wobei die Kaugummizusammensetzung 0,5 % bis 10 % der festen Peroxidzusammensetzung, bezogen auf das Gewicht der Gummizusammensetzung, umfasst.

13. Verfahren nach Anspruch 2, wobei die Kaugummizusammensetzung **1** % bis 20 % der gekapselten Peroxidzusammensetzung, bezogen auf das Gewicht der Gummizusammensetzung, umfasst.

14. Verfahren nach Anspruch 2, wobei die Gummizusammensetzung ferner einen Chelatbildner umfasst, der aus der Gruppe ausgewählt ist, die aus Polyphosphaten, Pyrophosphaten, Natriumtripolyphosphat, Polyphosphonaten und Kombinationen davon besteht.

15. Zahnweißkaugummizusammensetzung nach Anspruch 1, die umfasst:
(a) eine Kaugummibasis; und
(b) eine Vielzahl aktivstoffhaltiger Partikel, wobei jedes der aktivstoffhaltigen Partikel eine feste Peroxidzusammensetzung in einer Polymerkapselungsmatrix umfasst.

16. Zusammensetzung nach Anspruch 15, wobei das aktivstoffhaltige Partikel ferner eine Beschichtung aus Ethylzellulose umfasst.

17. Verfahren zum Weißen der Zähne eines Nutzers nach Anspruch 2, wobei das Verfahren die folgenden Schritte umfasst:
(a) Zubereiten einer Zahnweißkaugummizusammensetzung, wobei das Zubereiten die folgenden Schritte umfasst:
(i) Bereitstellen einer Kaugummibasis;
(ii) getrenntes Bereitstellen einer Vielzahl aktivstoffhaltiger Partikel, wobei die Partikel durch die folgenden Schritte zubereitet werden:
(1) Mischen eines Polymermaterials und einer festen Peroxidzusammensetzung, um ein Gemisch bereitzustellen;
(2) Extrudieren des Gemischs;
(3) Kühlen des Gemischs; und
(4) Mahlen des gekühlten Gemischs, um eine Vielzahl aktivstoffhaltiger Partikel bereitzustellen;
(iii) Kombinieren der Kaugummibasis und der Vielzahl aktivstoffhaltiger Partikel, um eine resultierende Kaugummizusammensetzung bereitzustellen;
(b) Bereitstellen der Kaugummizusammensetzung für einen Nutzer; und
(c) wobei der Nutzer die Kaugummizusammensetzung kaut, wobei das Kauen die feste Peroxidzusammensetzung freisetzt und dadurch die Zähne des Nutzers der Peroxidzusammensetzung aussetzt.

## Revendications

1. Composition de gomme à mâcher blanchissant les dents, comprenant :
(a) une base de gomme à mâcher et
(b) une pluralité de particules contenant une substance active, chacune des particules contenant une substance active comprenant une région intérieure comprenant une composition de peroxyde solide, une région médiane comprenant une première matière d'enrobage et une région extérieure comprenant une deuxième matière d'enrobage,
dans laquelle la première matière d'enrobage comprend du poly(acétate de vinyle) et la deuxième matière d'enrobage comprend une matière cellulosique et
chacune des particules contenant une substance active comprend entre 20 et 50% en poids de matière d'enrobage et entre 50 et 80% en poids de peroxyde.

2. Procédé de blanchiment des dents d'un utilisateur, comprenant les stades dans lesquels :
(a) on prépare une composition de gomme à mâcher blanchissant les dents, comprenant :
(i) une base de gomme à mâcher et
(ii) une pluralité de particules contenant une substance active, chacune des particules contenant une substance active comprenant une région intérieure comprenant une composition de peroxyde solide, une région médiane comprenant une première matière d'enrobage et une région extérieure comprenant une deuxième matière d'enrobage, dans laquelle la première matière d'enrobage comprend du poly(acétate de vinyle) et la deuxième matière d'enrobage comprend une matière cellulosique et chacune des particules contenant une substance active comprend entre 20 et 50% en poids de matière d'enrobage et entre 50 et 80% en poids de peroxyde;
(b) on procure la composition de gomme à mâcher à un utilisateur et
(c) l'utilisateur mâche la composition de gomme à mâcher, la mastication brisant la région extérieure et libérant la région intérieure dans la bouche, en soumettant ainsi les dents de l'utilisateur à la composition de peroxyde.

3. Composition de gomme suivant la revendication 1 ou procédé suivant la revendication 2, dans laquelle la composition de peroxyde solide est choisie dans le groupe consistant en le peroxyde de carbamide, le percarbonate de sodium, la peroxydone, le peroxyde de calcium, l'acide perbenzoïque et leurs combinaisons.

4. Composition de gomme suivant la revendication 1 ou procédé suivant la revendication 2, dans laquelle la région extérieure comprend une couche d'au moins une matière d'enrobage choisie dans le groupe consistant en de l'éthylcellulose, du poly(acétate de vinyle), du shellac, une cire, une matière grasse, un lipide, un émulsionnant, de la gomme arabique, du mannitol, de l'HPMC, du polyéthylène glycol, de la gélatine et leurs combinaisons.

5. Composition de gomme suivant la revendication 1, dans laquelle dans chacune des particules contenant une substance active, la région extérieure représente entre 10 et 50% du poids de la particule et la région intérieure entre 50 et 90% du poids de la particule.

6. Composition de gomme suivant la revendication 1, dans laquelle, dans la composition de gomme à mâcher, la composition de peroxyde solide représente de 0,5% à 6% du poids de la composition de gomme.

7. Composition de gomme suivant la revendication 1, dans laquelle, dans la composition de gomme à mâcher, la particule de peroxyde enrobée représente de 0,5% à 25% du poids de la composition de gomme.

8. Composition de gomme suivant la revendication 1 ou procédé suivant la revendication 2, dans laquelle chacune des particules contenant une substance active comprend au moins deux matières distinctes d'enrobage.

9. Composition de gomme ou procédé suivant la revendication 8, dans laquelle chacune des particules contenant une substance active comprend la région intérieure, comprenant une composition de peroxyde solide, une première région extérieure entourant la région intérieure et une deuxième région extérieure entourant la première région extérieure, la première et la deuxième régions extérieures comprenant des matières d'enrobage différentes.

10. Composition de gomme ou procédé suivant la revendication 9, dans laquelle la première région extérieure comprend une matière hydrophobe et la deuxième région extérieure comprend une matière hydrophile.

11. Procédé suivant la revendication 2, dans lequel chacune des particules contenant une substance active comprend une région extérieure représentant entre 20 et 50% du poids de la particule et une région intérieure représentant entre 50 et 80% du poids de la particule.

12. Composition de gomme ou procédé suivant la revendication 2, dans laquelle, dans la composition de gomme à mâcher, la composition de peroxyde solide représente de 0,5 à 10% du poids de la composition de gomme.

13. Composition de gomme ou procédé suivant la revendication 2, dans laquelle, dans la composition de gomme à mâcher, la composition de peroxyde enrobé représente de 1% à 20% du poids de la composition de gomme.

14. Composition de gomme ou procédé suivant la revendication 2, dans laquelle la composition de gomme comprend, en outre, un agent de chélation choisi dans le groupe consistant en les polyphosphates, les pyrophosphates, le tripolyphosphate de sodium, les polyphosphonates et leurs combinaisons.

15. Composition de gomme à mâcher blanchissant les dents suivant la revendication 1, comprenant :
(a) une base de gomme à mâcher et
(b) une pluralité de particules contenant une substance active, chacune des particules contenant une substance active comprenant une composition de peroxyde solide au sein d'une matrice polymère d'enrobage.

16. Composition suivant la revendication 15, dans laquelle la particule contenant une substance active comprend, en outre, un enrobage d'éthyle cellulose.

17. Procédé de blanchiment des dents d'un utilisateur suivant la revendication 2, comprenant les stades de :
(a) préparer une composition de gomme à mâcher blanchissant les dents, comprenant les stades dans lesquels :
(i) on se procure une base de gomme à mâcher;
(ii) on se procure séparément une pluralité de particules contenant une substance active préparées par les stades dans lesquels :
(1) on mélange une matière polymère et une composition de peroxyde solide pour obtenir un mélange;
(2) on extrude le mélange;
(3) on refroidit le mélange et
(4) on broie le mélange refroidi pour obtenir une pluralité de particules contenant une substance active;
(iii)on combine la base de gomme à mâcher et la pluralité de particules contenant une substance active pour obtenir une composition résultante de gomme à mâcher,
(b) procurer la composition de gomme à mâcher à un utilisateur et
(c) l'utilisateur mastiquant la composition de gomme à mâcher, la mastication libérant la composition de peroxyde solide en soumettant ainsi les dents de l'utilisateur à la composition de peroxyde.
